# EUROPEAN PATENT APPLICATION

(11) **EP 4 826 512 A1**
(43) Date of publication of application: **21.10.2026**
(21) Application number: 26171648.4
(22) Date of filing: 10.04.2026
(51) Int. Cl.: G06F 3/12, G06F 21/60, G16H 10/60, H04W 12/06, H04W 12/47, H04W 12/71

(54) **ELECTRONIC CONSENT DOCUMENT SYSTEM**

(30) Priority: 15.04.2025 JP 2025066655
(71) Applicant: KYOCERA Document Solutions Inc., Osaka-shi, Osaka 540-8585 (JP)
(72) Inventor: FUJIMORI, Taiki, Osaka, 540-0004 (JP); HARADA, Eriko, Osaka, 540-0004 (JP); HIRANO, Masayoshi, Osaka, 540-0004 (JP); NOGAWA, Hiroaki, Osaka, 540-0004 (JP)
(74) Representative: Winter, Brandl - Partnerschaft mbB

(57) **Abstract**

A terminal apparatus is a portable terminal apparatus, displays an electronic consent form, acquires signature data, and transmits to a management server electronic consent document data that includes the signature data such that the electronic consent document data is associated with a device ID of this terminal apparatus. When receiving the electronic consent document data, the management server transmits a print job based on the electronic consent document data such that the print job is associated with the device ID. A print server spools the print job. Further, when detecting the device ID, the image forming apparatus transmits a transmission request corresponding to the detected device ID to the print server, the print server transmits a print job corresponding to the device ID specified by the transmission request, and the image forming apparatus receives and performs the print job corresponding to the detected device ID.

## Description

### BACKGROUND

### 1. FIELD OF THE PRESENT INVENTION

The present invention relates to an electronic consent document system.

### 2. DESCRIPTION OF THE RELATED ART

In an information processing system, consent form data is displayed on a terminal apparatus of a patient, and when a signature of the patient is electronically inputted to the consent form data, the consent form data that the input information such as the signature has been applied is displayed on the terminal apparatus, and thereafter, pressing down a "consent" button causes the signature process to be finished, and thereafter, the consent form data is submitted to a server.

However, in the aforementioned information processing system, when plural consent documents are printed, a wrong consent document may be taken from the plural consent documents.

### SUMMARY

An electronic consent document system according to the present invention includes a management server connected to a network, a terminal apparatus connected to the network, a print server connected to the network, and an image forming apparatus. The terminal apparatus is a portable terminal apparatus, displays an electronic consent form of a specific matter, acquires signature data on the basis of a signature operation to the electronic consent form, and transmits to the management server electronic consent document data that includes the signature data corresponding to the electronic consent form such that the electronic consent document data is associated with a device ID of this terminal apparatus. When the management server receives the electronic consent document data from the terminal apparatus, the management server transmits a print job based on the electronic consent document data to the print server such that the print job is associated with the device ID of the terminal apparatus. When the print server receives the print job, the print server spools the print job. Further, the image forming apparatus (a) includes a device ID reader that reads the device ID of the terminal apparatus from the terminal apparatus, (b) when the device ID is detected by the device ID reader, transmits a transmission request for a print job corresponding to the detected device ID to the print server, and (c) receives the print job corresponding to the detected device ID from the print server and performs the print job. Meanwhile, when the print server receives the transmission request, the print server transmits one or plural print jobs corresponding to the device ID specified by the transmission request among the spooled print jobs.

These and other objects, features and advantages of the present invention will become more apparent upon reading of the following detailed description along with the accompanied drawings.

### BRIEF DESCRIPTION OF THE DRAWINGS

Fig. 1 shows a block diagram that indicates a configuration of an electronic consent document system according to an embodiment of the present invention;
Fig. 2 shows a diagram that indicates a display example of an explanation document image;
Fig. 3 shows a diagram that indicates a display example of an electronic consent form after date input and handwriting signature;
Fig. 4 shows a diagram that indicates a display example of an electronic consent form (confirmation image);
Fig. 5 shows a diagram that indicates an example of the printed electronic consent document;
Fig. 6 shows a sequence diagram that explains a behavior of the electronic consent document system shown in Fig. 1; and
Fig. 7 shows a diagram that explains a flow line of a healthcare worker when the healthcare worker wants plural patients to sign electronic consent forms by handwriting in their patient rooms, respectively.

### DETAILED DESCRIPTION

Hereinafter, an embodiment according to an aspect of the present invention will be explained with reference to drawings.

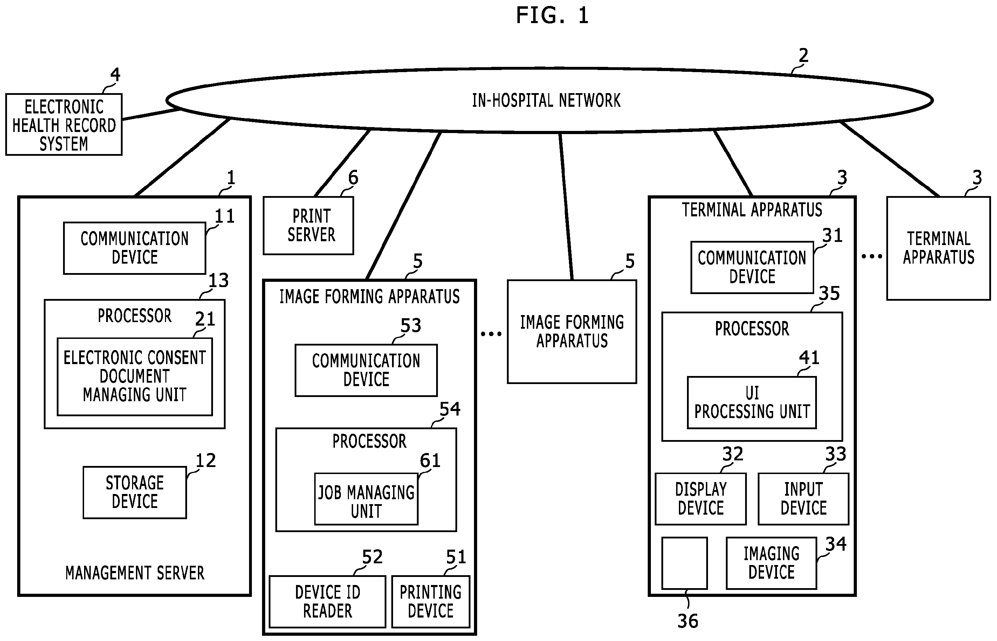

Fig. 1 shows a block diagram that indicates a configuration of an electronic consent document system according to an embodiment of the present invention. In the electronic consent document system shown in Fig. 1, a management server 1 is connected to an in-hospital network 2, and to the in-hospital network 2, one or plural terminal apparatuses 3, an electronic health record system 4, one or plural image forming apparatuses 5, and a print server 6 are connected.

The in-hospital network 2 is a local area network in a hospital, a clinic or the like, and the management server 1 is an on-premise server apparatus that includes a computer. Each of the terminal apparatuses 3 is connected to the in-hospital network 2 through wireless or wired communication, is carried by a healthcare worker, and presents an explanation document, an electronic consent form, and the like when explaining a specific matter (surgery or the like) to a patient. The terminal apparatus 3 is a personal computer or a portable terminal such as a tablet terminal or a smart phone.

The electronic health record system 4 is a system that manages an electronic health record with an on-premise server apparatus, and enables the terminal apparatus connected to the in-hospital network 2 to perform generation of a new electronic health record, editing of an electronic health record, viewing of an electronic health record and the like.

Each image forming apparatus 5 is an apparatus such as printer or multi function peripheral, that has a printing function. Here, the image forming apparatus 5 acquires a print job (print image data, print settings and the like) from the print server 6 through the in-hospital network 2, but alternatively, the image forming apparatus 5 may be directly connected to the print server 6 with a peripheral device interface (USB or the like) and may acquire a print job from the print server 6 not through the in-hospital network 2. The print server 6 receives and spools a print job from the management server 1 and transmits the print job to the image forming apparatus 5.

As mentioned, this electronic consent document system is an on-premise system, and therefore, easily cooperates with the existing on-premise electronic health record system 4.

Further, the terminal apparatus 3 is a portable terminal apparatus (tablet terminal or the like), and the terminal apparatuses 3 are assigned to plural healthcare workers; and one of the image forming apparatuses 5 is arranged in a nurse station used by the plural healthcare workers.

Further, the management server 1 includes a communication device 11, a storage device 12, and a processor 13. The communication device 11 is a device such as a network interface connected to the in-hospital network 2. The storage device 12 is a non-volatile storage device such as flash memory, and stores data and/or a program used by the processor 13. The processor 13 includes a computer and the computer includes a CPU (Central Processing Unit), a ROM (Read Only Memory), a RAM (Random Access Memory) and the like, loads a program stored in the ROM or the storage device 12 to the RAM, executes the program with the CPU, and thereby acts as sorts of processing units. Here, the computer act as an electronic consent document managing unit 21.

Meanwhile, each of the terminal apparatuses 3 includes a communication device 31, a display device 32, an input device 33, an imaging device 34, and a processor 35. Further, in this embodiment, the terminal apparatus 3 includes a device ID unit 36 from which the image forming apparatus 5 is enabled to read a device ID of this terminal apparatus 3. The device ID is data unique to the terminal apparatus 3 (i.e. identification information). In this embodiment, the device ID unit 36 is an RFID (Radio Frequency Identification) tag.

The communication device 31 is a wired or wireless network interface or the like connected to the in-hospital network 2.

The display device 32 includes a liquid crystal display panel or the like and displays a view screen, an operation screen and the like. The input device 33 includes a touch panel, a hard key and/or the like and detects a user operation. For example, a touch panel as the input device 33 is arranged on a display panel of the display device 32, and the display device 32 displays an image of a soft key at predetermined area and the input device 33 detects a user operation to the area and thereby the soft key is formed.

The imaging device 34 includes an imaging sensor, and takes and generates an image (image data) of a scene in front of a housing that the display device 32 and the input device 33 are arranged (e.g. an image of a user who operates this terminal apparatus 3 or the like).

The processor 35 includes a computer and the computer includes a CPU, a ROM, a RAM and the like, and loads a program stored in the ROM or the like to the RAM and executes the program with the CPU and thereby acts as processing units. Here, the computer acts as a user interface processing unit 41.

Further, the user interface processing unit 41 in the terminal apparatus 3 displays an electronic consent form of a specific matter (e.g. medical procedure such as surgery) on the display device 32, acquires signature data using the input device 33 on the basis of a signature operation to the electronic consent form, and transmits using the communication device 31 to the management server 3 electronic consent document data that includes the signature data corresponding to the electronic consent form such that the electronic consent document data is associated with the device ID of this terminal apparatus 3.

The electronic consent document data may be a data set that includes the signature data and the electronic consent form, or the electronic consent document data may be raster image data obtained by synthesizing the signature data and the electronic consent form.

Meanwhile, the electronic consent document managing unit 21 in the management server 1 receives the electronic consent document data from the terminal apparatus 3 through the in-hospital network 2 using the communication device 11, and when receiving the electronic consent document data, the electronic consent document managing unit 21 transmits a print job of an electronic consent document based on the electronic consent document data to the print server 6 such that the print job is associated with the device ID; when receiving the print job, the print server 6 spools the print job such that the print job is associated with the device ID; and when receiving a transmission request that specifies a device ID, the print server 6 transmits one or plural print jobs associated with the device ID to the image forming apparatus 5 and thereby causes the image forming apparatus 5 to print the electronic consent document(s) on the basis of the print job(s).

In this embodiment, the aforementioned signature data is raster image data of a handwriting signature. Specifically, handwriting stokes inputted with a stylus pen or the like are detected by the input device 33 (touch panel), and data of an image that includes the handwriting stokes is generated as the signature data.

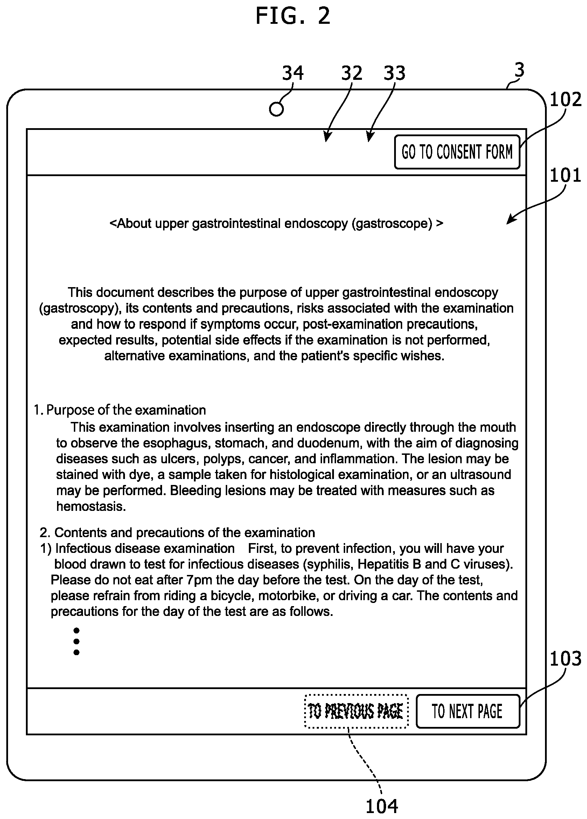

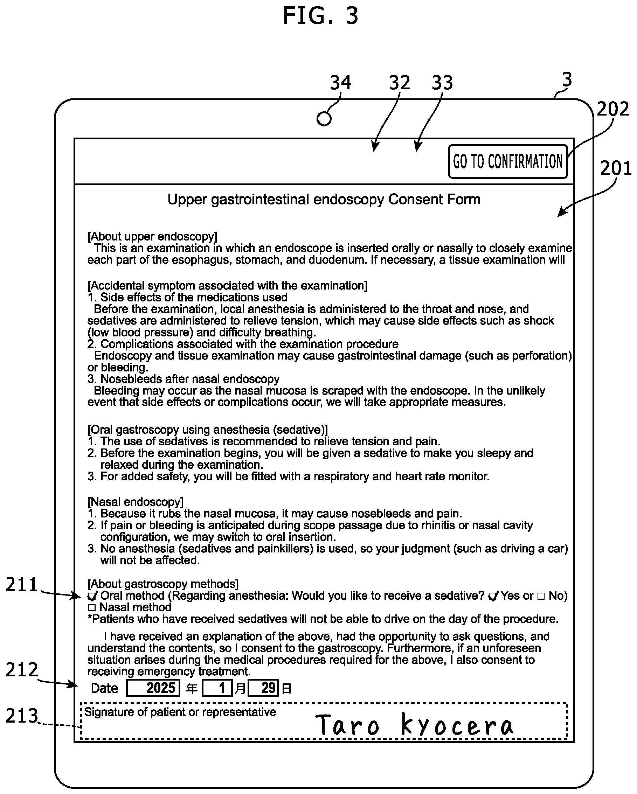

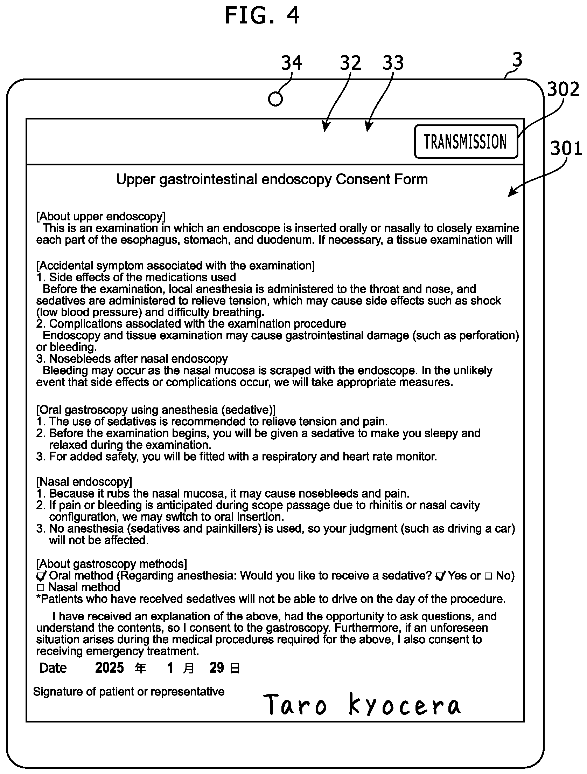

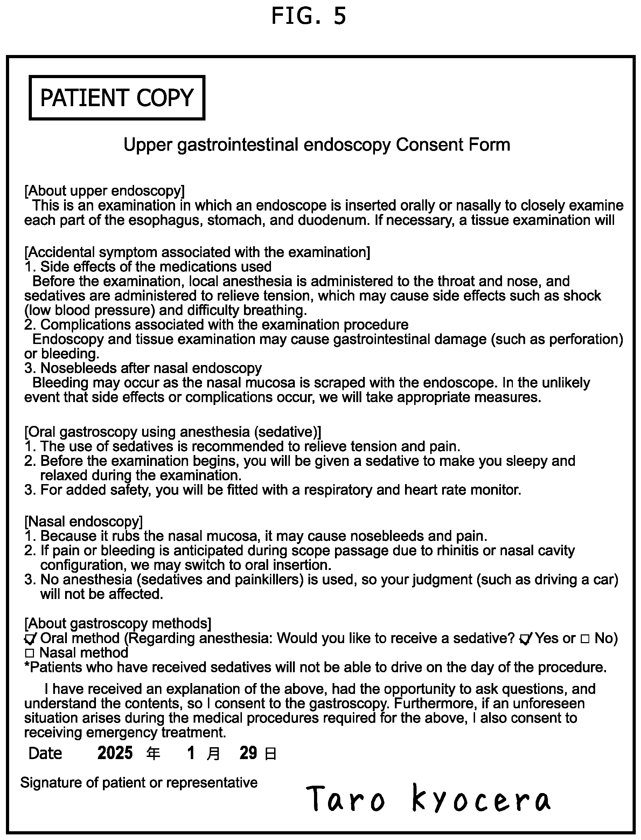

Fig. 2 shows a diagram that indicates a display example of an explanation document image. Fig. 3 shows a diagram that indicates a display example of an electronic consent form after date input and handwriting signature. Fig. 4 shows a diagram that indicates a display example of an electronic consent form (confirmation image). Fig. 5 shows a diagram that indicates an example of the printed electronic consent document.

Further, in this embodiment, the user interface processing unit 41 in the terminal apparatus 3 (a) displays an explanation document image of the aforementioned specific matter on the display device 32 as shown in Fig. 2 for example, (b) displays an electronic consent form of the specific matter after displaying the explanation document image on the display device 32 as shown in Fig. 3 for example, (c) displays an electronic consent document (i.e. confirmation image) based on the electronic consent document data after detecting the aforementioned signature operation using the input device 33 on the display device 32 as shown in Fig. 4 for example, and (d) transmits the electronic consent document data to the management server 1 using the communication device 31 after detecting a predetermined confirmation operation using the input device 33. Consequently, the signed electronic consent document is printed on a print paper sheet or the like as shown in Fig. 5, for example. The printed signed electronic consent document is handed over to a patient as a patient copy.

As shown in Fig. 2 for example, in a display screen of the explanation document image 101, soft keys 102, 103 and 104 are displayed together with the explanation document image 101. The soft key 102 is a soft key to change a screen to a display screen of the electronic consent form, the soft key 103 is a soft key to change a displayed page to a next page among plural pages in the explanation document image 101, and the soft key 104 is a soft key to change a displayed page to a previous page among plural pages in the explanation document image 101.

For example, the soft key 102 may be displayed so as to be operatable after all the pages of the explanation document image 101 are displayed, and before all the pages of the explanation document image 101 are displayed, the soft key 102 may be grayed out.

Further, when the soft key 102 is pressed down, the user interface processing unit 41 changes a screen to a display screen of the electronic consent form. As shown in Fig. 3 for example, in the display screen of the electronic consent form, the electronic consent form 201 as a document image and a soft key 202 are displayed. The soft key 202 is a soft key to change a screen to a confirmation screen of the electronic consent document.

The electronic consent form 201 includes a check box 211, a signature date input field 212, and a handwriting signature area 213. In the check box 211, a check image is displayed correspondingly to a check operation. The signature date input field 212 is pull-down lists; a year, a month and a day of the signature date are selected from the pull-down lists in accordance with user operations; and the selected year, the selected month and the selected day are displayed in the signature date input field 212. In the handwriting signature area 213, handwriting strokes of handwriting signature input are acquired, and a handwriting signature image is displayed correspondingly to the handwriting strokes.

The soft key 202 may be displayed so as to be operatable after the handwriting signature image is acquired, and therebefore the soft key 202 may be grayed out.

Further, when the soft key 202 is pressed down, the user interface processing unit 41 changes a screen to a confirmation screen of the electronic consent document. As shown in Fig. 4 for example, in the confirmation screen, an electronic consent document image 301 and a transmission key 302 are displayed. The transmission key 302 is a soft key to transmit the electronic consent document data to the management server 1. When the transmission key 302 is pressed down, the user interface processing unit 41 transmits the electronic consent document data to the management server 1 using the communication device 31.

Further, the terminal apparatus 3 (a) may detect an additionally-writing operation performed by a healthcare worker onto an explanation document image and generate an additionally-writing image based on the additionally-writing operation, (b) may generate a signature image based on the signature operation, and (c) may include the explanation document image, the additionally-writing image, the electronic consent form, and the signature image into the electronic consent document data. In this case, for example, the additionally-writing image and the signature image are included into the electronic consent document data such that the additionally-writing image and the signature image are separated from each other (e.g. as handwriting strokes with different colors from each other).

Further, using the imaging device 34, the terminal apparatus 3 may acquire a movie of a healthcare worker in a period that the healthcare worker explains the specific matter on the basis of the explanation document image (i.e. in a period that the explanation document image is displayed), and may include movie data of the movie into the electronic consent document data. Further, a sound correction device may be installed in the terminal apparatus 3, and using the sound correction device, the terminal apparatus 3 may acquire a voice of a healthcare worker in a period that the healthcare worker explains the specific matter on the basis of the explanation document image, and may include voice data of the voice into the electronic consent document data. Consequently, at a later date, playing the movie data or the voice data in the electronic consent document data enables to verify whether the explanation was properly performed or not.

Furthermore, (a) when the aforementioned signature operation is detected, the user interface processing unit 41 in the terminal apparatus 3 may take and generate an image of a person who performs the signature operation using the imaging device 34, and (b) the user interface processing unit 41 may include the taken image into the electronic consent document data. Consequently, at a later date, displaying the taken image in the electronic consent document data enables to verify whether a person who should sign (patient or the like) properly signed or not.

Further, the user interface processing unit 41 in the terminal apparatus 3 displays the explanation document and the electronic consent form in an page order on the basis of a single file (e.g. PDF (Portable Document Format) file) generated in the page order of 1) the explanation document, 2) the electronic consent form; and the electronic consent document managing unit 21 in the management server 1 generates as an electronic consent document for storage a single file generated in a page order of 1) the electronic consent form, 2) the explanation document (i.e. in a reverse order of the displaying page order) together with the signature data on the basis of the electronic consent document data, and automatically registers the electronic consent document for storage to the electronic health record system 4 such that the electronic consent document for storage can be viewed. Consequently, the patient easily signs the electronic consent form after reading the explanation document, and at a later date, when the healthcare worker wants to confirm the signed electronic consent document, the healthcare worker can immediately see the signed electronic consent document.

Further, when the electronic consent document managing unit 21 in the management server 1 receives the electronic consent document data from any of the terminal apparatuses 3, the electronic consent document managing unit 21 may immediately transmit the print job to the print server 6 and automatically register either the electronic consent document data or electronic consent document for storage (e.g. raster image data of it) based on the electronic consent document data into the electronic health record system 4.

Furthermore, at that time, after finishing printing of the electronic consent document based on the print job, the electronic consent document managing unit 21 in the management server 1 may automatically register the electronic consent document data or electronic consent document for storage based on the electronic consent document data into the electronic health record system 4.

Furthermore, when a single patient is asked to sign plural electronic consent documents of plural specific matters, the user interface processing unit 41 in the terminal apparatus 3 may display plural electronic consent forms of plural specific matters on the display device 32, detect a signature operation to a bulk signature form corresponding to the plural electronic consent forms using the input device 33, acquire signature data based on the signature operation, and transmit to the management server 1 plural pieces of the electronic consent document data corresponding to the plural electronic consent forms such that each of the plural pieces of the electronic consent document data includes the signature data. Consequently, only one signature operation is sufficient for plural electronic consent forms, and therefore, a patient convenience is gained.

Meanwhile, each image forming apparatus 5 includes a printing device 51, a device ID reader 52, a communication device 53, and a processor 54.

The printing device 51 prints on a print sheet an image based on a print job in accordance with a predetermined printing manner (an electrographic manner, an inkjet manner or the like).

The device ID reader 52 is a reader device that reads a device ID of the terminal apparatus 3. For example, if the device ID is indicated by the RFID tag as mentioned, an RFID reader is used as the device ID reader 52.

The communication device 53 is a wired or wireless interface (network interface or peripheral device interface) capable of communication with the print server 6.

The processor 54 includes a computer and the computer includes a CPU, a ROM, a RAM and the like, and loads a program stored in the ROM, a storage device (not shown) or the like to the RAM and executes the program with the CPU and thereby acts as processing units. Here, the computer acts as a job managing unit 61.

When the device ID reader 52 detects a device ID of the terminal apparatus 3, the job managing unit 61 transmits to the print server 6 a transmission request of a print job corresponding to the detected device ID, and receives one or plural print jobs corresponding to the detected device ID and immediately performs the received print job(s) using the print device 51. Consequently, one or plural print products of one or plural electronic consent documents is/are provided by the print device 51.

At that time, when receiving the transmission request, the print server 6 selects one or plural print jobs corresponding to the device ID specified by the transmission request among the spooled print jobs and transmits the selected print job(s) to the image forming apparatus 5 that transmitted the transmission request.

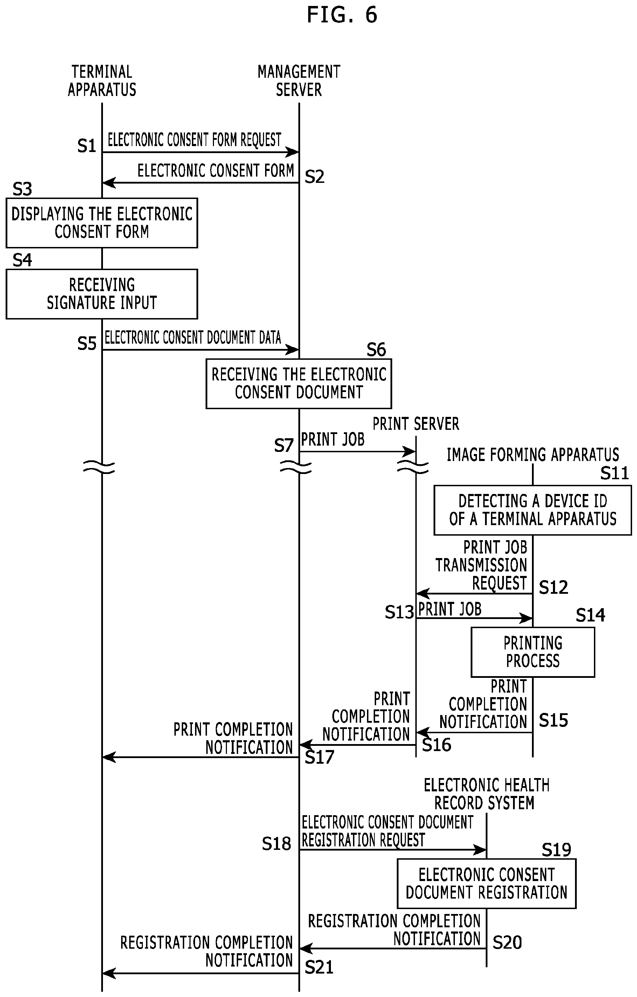

The following part explains a behavior of the aforementioned electronic consent document system. Fig. 6 shows a sequence diagram that explains a behavior of the electronic consent document system shown in Fig. 1.

When the user interface processing unit 41 in the terminal apparatus 3 detects a predetermined user operation using the input device 33, the user interface processing unit 41 transmits an electronic consent form request to the management server 1 (in Step S1). When the electronic consent document managing unit 21 in the management server 1 receives the electronic consent form request, the electronic consent document managing unit 21 transmits an electronic consent form to the terminal apparatus 3 (in Step S2).

For example, the user interface processing unit 41 includes a web client and the electronic consent document managing unit 21 includes a web server, and the user interface processing unit 41 transmits the electronic consent form request using the web client in accordance with HTTP (Hypertext Transfer Protocol). The electronic consent document managing unit 21 transmits the electronic consent form using the web server in accordance with HTTP.

When the user interface processing unit 41 in the terminal apparatus 3 receives the electronic consent form, the user interface processing unit 41 displays the electronic consent form on the display device 32 (in Step S3).

Here, as mentioned, a PDF file that includes the electronic consent form and the explanation document image is transmitted to the terminal apparatus 3, the electronic consent form and the explanation document image are displayed in turn, and after a handwriting signature operation is detected, a confirmation screen is displayed.

When an operation to the check box 211, an operation to the signature data input field 212, and a handwriting signature operation to the handwriting signature area 213 are detected by the input device 33 (in Step S4), the user interface processing unit 41 generates the electronic consent document data that includes a value of the check box 211 (i.e. checked or not), a value of the signature data input field 212 (i.e. year-month-day data), a handwriting signature image (raster image data), and the electronic consent form, and transmits the electronic consent document data such that the electronic consent document data is associated with the device ID of this terminal apparatus 3 (after the confirmation screen is displayed) (in Step S5).

When the electronic consent document managing unit 21 in the management server 1 receives the electronic consent document data (in Step S6), the electronic consent document managing unit 21 generates a print job (print image data and print settings) of the electronic consent document and transmits the print job to the print server 6 (in Step S7). The print server 6 receives the print job and spools the print job such that the print job is associated with the device ID of the terminal apparatus 3. Before generating the print job, the electronic consent document managing unit 21 may determine whether the signature data is included in the electronic consent document data or not, and if the signature data is not included, may terminate the process due to error.

As mentioned, every time that the electronic consent document data is obtained, a print job of an electronic consent document is additionally spooled in the print server 6 such that the print job is associated with the device ID of the terminal apparatus 3.

Afterward, in one of the image forming apparatuses 5, when the device ID of the terminal apparatus 3 is detected by the device ID reader 52 (in Step S11), the job managing unit 61 transmits a print job transmission request that specifies this device ID to the print server 6 using the communication device 53 (in Step S12).

When receiving the print job transmission request, among the spooled print jobs, the print server 6 selects one or plural print jobs associated with the device ID specified by the print job transmission request and transmits the selected print job(s) back to the image forming apparatus (in Step S13).

In this image forming apparatus 5, the job managing unit 61 receives the print job(s) using the communication device 53 and immediately performs the print job(s) and thereby causes the printing device 51 to print one or plural images based on the print job(s) (here, one or plural electronic consent documents) based on the print job(s) (in Step S14). When finishing the print job, the job managing unit 61 transmits a print completion notification to the print server 6 using the communication device 53 (in Step S15). Here, if plural print jobs are received, the job managing unit 61 may transmit print completion notifications for the plural print jobs, respectively, or may transmit a single transmit print completion notification for the plural print jobs.

When receiving the print completion notification, the print server 6 transmits the print completion notification to the management server 1 (in Step S16), and receiving the print completion notification, the electronic consent document managing unit 21 in the management server 1 transmits the print completion notification to the terminal apparatus 3 (in Step S17). For example, when the user interface processing unit 41 in the terminal apparatus 3 receives the print completion notification, the user interface processing unit 41 displays a message that indicates print completion on the display device 32.

Further, when the electronic consent document managing unit 21 in the management server 1 receives the print completion notification, the electronic consent document managing unit 21 immediately transmits an electronic consent document registration request that includes the electronic consent document to the electronic health record system 4 correspondingly to the print completion notification (in Step S18).

When receiving the electronic consent document registration request, the electronic health record system 4 registers the electronic consent document for storage (in Step S19).

For example, the electronic consent form is acquired from the electronic health record system 4 together with a patient ID of a patient of which an electronic consent document should be acquired, and in this electronic consent document system, the electronic consent document data is acquired in association with the patient ID, and the electronic consent document data is registered in association with the patient ID in the electronic health record system 4 such that the electronic consent document data can be viewed. Consequently, in the electronic health record system 4, the electronic consent document of the patient with the patient ID can be viewed.

When finishing the registration of the electronic consent document, the electronic health record system 4 transmits a registration completion notification to the management server 1 (in Step S20); and when the electronic consent managing unit 21 in the management server 1 receives the registration completion notification, the electronic consent managing unit 21 transmits it to the terminal apparatus 3 (in Step S21). For example, when the user interface processing unit 41 in the terminal apparatus 3 receives the registration completion notification, the user interface processing unit 41 displays on the display device 32 a message that indicates registration completion of the electronic consent document to the electronic health record system 4. Further, after receiving the registration completion notification of the electronic consent document, the electronic health record system 4 or the electronic consent document managing unit 21 may immediately output an order for the aforementioned specific matter (i.e. instruction for performing the specific matter).

As mentioned, in the electronic consent document system according to the aforementioned embodiment, the terminal apparatus 3 displays an electronic consent form of a specific matter, acquires signature data on the basis of a signature operation to the electronic consent form, and transmits to the management server 1 electronic consent document data that includes the signature data corresponding to the electronic consent form such that the electronic consent document data is associated with a device ID of this terminal apparatus 3. When the management server 1 receives the electronic consent document data from the terminal apparatus 3, the management server 1 transmits a print job based on the electronic consent document data to the print server 6 such that the print job is associated with the device ID of the terminal apparatus 3; and the print server 6 receives and spools the print job. Further, the image forming apparatus 5 (a) includes the device ID reader 52 that reads the device ID of the terminal apparatus 3 from the terminal apparatus 3, (b) when the device ID is detected by the device ID reader 52, transmits a transmission request for a print job corresponding to the detected device ID to the print server 6, and (c) receives the print job corresponding to the detected device ID from the print server 6 and performs the received print job. Meanwhile, when receiving the transmission request, the print server 6 selects one or plural print jobs corresponding to the device ID specified by the transmission request among the spooled print jobs and transmits the selected print job(s) to the image forming apparatus 5.

Consequently, an electronic consent document is printed such that a print job of the electronic consent document is associated with the device ID of the terminal apparatus 3 that transmitted the electronic consent document data, and therefore, it is restrained that a wrong consent document is taken from plural consent documents.

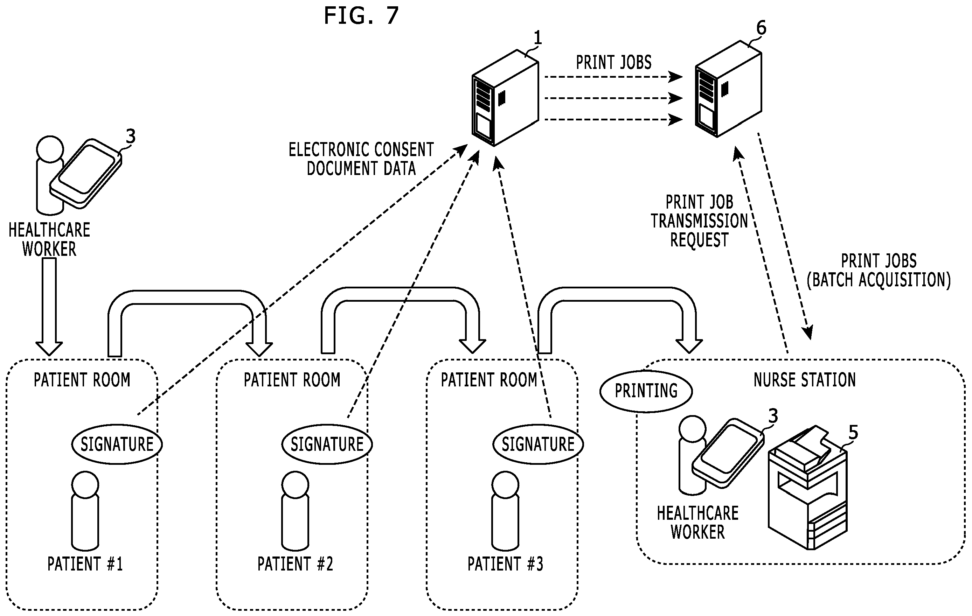

Fig. 7 shows a diagram that explains a flow line of a healthcare worker when the healthcare worker wants plural patients to sign electronic consent forms by handwriting in their patient rooms, respectively. In a case that a healthcare worker wants plural patients to sign electronic consent forms by handwriting in their patient rooms, as shown in Fig. 7 for example, the healthcare worker goes to the patient rooms in turn and causes the patients to sign electronic consent forms by handwriting, and thereafter, goes to a place such as nurse station that the image forming apparatus 5 is arranged and causes the image forming apparatus 5 to read the device ID of the terminal apparatus 3; and thereby, electronic consent documents (print products) that have been signed by handwriting are obtained in bulk (at once). Therefore, workload of the healthcare worker is reduced. Further, the electronic consent document is not printed before the image forming apparatus 5 reads the device ID of the terminal apparatus 3 of this healthcare worker, and therefore, information leakage and lost of the print product as the electronic consent document are restrained.

It should be understood that various changes and modifications to the embodiments described herein will be apparent to those skilled in the art. Such changes and modifications may be made without departing from the spirit and scope of the present subject matter and without diminishing its intended advantages. It is therefore intended that such changes and modifications be covered by the appended claims.

For example, in the aforementioned embodiment, the check box 211 in the electronic consent form 201 may be displayed with different colors before and after being checked. Consequently, forgetting to check is restrained.

Further, in the aforementioned embodiment, the RFID tag is used as the device ID of the terminal apparatus 3, but alternatively, a visible code that has the device ID may be used, such as bar code or a 2-dimensional code. In such a case, the visible code may be attached as a sheet-shaped member on a housing of the terminal apparatus 3, or may be displayed on the display device 32 by the user interface processing unit 41 when required (when causing the image forming apparatus 5 to read it, or the like). In such a case, the device ID reader 52 includes an imaging element and a decoder for the visible code.

Furthermore, in the aforementioned embodiment, the management server 1 is connected to the in-hospital network 2, but alternatively, the management server 1 may be connected to a global network that the in-hospital network 2 is connected.

## Claims

1. An electronic consent document system, comprising:
a management server (1) connected to a network (2);
a terminal apparatus (3) connected to the network (2);
a print server (6) connected to the network (2); and
an image forming apparatus (5);
wherein the terminal apparatus (3) is a portable terminal apparatus, displays an electronic consent form of a specific matter, acquires signature data on the basis of a signature operation to the electronic consent form, and transmits to the management server (1) electronic consent document data that includes the signature data corresponding to the electronic consent form such that the electronic consent document data is associated with a device ID of this terminal apparatus;
when the management server (1) receives the electronic consent document data from the terminal apparatus (3), the management server (1) transmits a print job based on the electronic consent document data to the print server such that the print job is associated with the device ID of the terminal apparatus;
when the print server (6) receives the print job, the print server (6) spools the print job;
the image forming apparatus (5) comprises a device ID reader (52) that reads the device ID of the terminal apparatus (3) from the terminal apparatus (3), when the device ID is detected by the device ID reader (52), transmits a transmission request for a print job corresponding to the detected device ID to the print server (6), and receives the print job corresponding to the detected device ID from the print server (6) and performs the print job; and
when the print server (6) receives the transmission request, the print server (6) transmits one or plural print jobs corresponding to the device ID specified by the transmission request among the spooled print jobs.

2. The electronic consent document system according to claim 1, wherein the terminal apparatus (3) comprises an RFID tag that has the device ID; and
the device ID reader (52) reads the device ID from the RFID tag.

3. The electronic consent document system according to claim 1, wherein the signature data is raster image data of a handwriting signature.

4. The electronic consent document system according to claim 1, wherein when the management server (1) receives the electronic consent document data, the management server (1) immediately transmits the print job to the print server (6) and automatically registers to an electronic health record system (4) either the electronic consent document data or an electronic consent document for storage based on the electronic consent document data.

5. The electronic consent document system according to claim 4, wherein after printing of the electronic consent document based on the print job is finished, the management server (1) automatically registers to an electronic health record system (4) either the electronic consent document data or an electronic consent document for storage based on the electronic consent document data.
